(19) Europäisches Patentamt
European Patent Office
Office européen des brevets

(11) Publication number: 0 510 473 A1

(12) **EUROPEAN PATENT APPLICATION**

(21) Application number: 92106339.2

(22) Date of filing: 13.04.92

(51) Int. Cl.[5]: C07D 333/32, A61K 31/38

(30) Priority: 24.04.91 US 690476

(43) Date of publication of application: 28.10.92 Bulletin 92/44

(84) Designated Contracting States: AT BE CH DE DK ES FR GB GR IT LI LU MC NL PT SE

(71) Applicant: F. HOFFMANN-LA ROCHE AG
Postfach 3255
CH-4002 Basel(CH)

(72) Inventor: Coffey, John William
152 Washington Avenue
West Caldwell, N.J. 07006(US)
Inventor: Lovey, Allen John
49 Park Avenue
Bloomfield, N.J. 07003(US)
Inventor: Rosenberger, Michael
79 Arlington Avenue
Caldwell, N.J. 07006(US)

(74) Representative: Grossner, Lutz, Dr. et al
Grenzacher Strasse 124 Postfach 3255
CH-4002 Basel(CH)

# (54) Thienyl-nonatetraenoic acid derivatives with anti-rheumatic activity.

(57) Novel 9-thienyl-3,7-dimethyl-2,4,6,8-nonatetraenoic acids wherein the thiophene group is substituted with an alkyl and alkoxy group, and derivatives thereof can be used as disease modifying anti-rheumatic agents and immunosuppressants.

EP 0 510 473 A1

The present invention relates to novel thienylnonatetraenoic acid derivatives of the formula

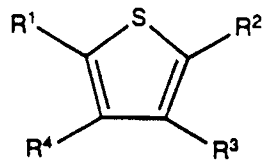

I

wherein either

$R^1$ is hydrogen or lower alkyl,
$R^2$ is a moiety Z,
$R^3$ is $-O(CH_2)_nCH_3$, and
$R^4$ is hydrogen; or
$R^1$ and $R^2$ are independently hydrogen or lower alkyl,
$R^3$ is a moiety Z, and
$R^4$ is $-O(CH_2)_nCH_3$; or
$R^1$ is $-O(CH_2)_nCH_3$,
$R^2$ is a moiety Z, and
$R^3$ and $R^4$ are independently hydrogen or lower alkyl; Z is a moiety

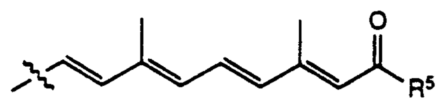

$R^5$ is hydrogen, lower alkyl, amino or N-lower alkylamino; and n is an integer from 3 to 8;
and salts of compounds of formula I wherein $R^5$ is hydrogen.

The invention also relates to a process for the preparation of the compounds of formula I, pharmaceutical preparations containing these compounds and the use of these compounds in the preparation of pharmaceutical preparations for the treatment of psoriatic and rheumatoid arthritis.

Also included in this invention are salts of the compounds of formula I above, with pharmaceutically acceptable, non-toxic, inorganic or organic bases, e.g., alkali metal and alkaline earth metal salts. Among the preferred salts are the sodium, potassium, magnesium or calcium salts, as well as salts with ammonia or suitable non-toxic amines, such as lower alkyl amines, for example, triethylamine, hydroxy-lower alkylamines, for example, 2-hydroxyethylamine, bis-(1-hydroxyethyl)-amine or tris-(2-hydroxyethyl)-amine, cycloalkylamines, for example, dicyclohexylamine, or benzylamines, for example, N,N'-dibenzylethylenediamine and dibenzylamine. These salts can be prepared by treating the compounds of formula I, where $R^5$ is hydrogen with inorganic or organic bases by conventional means well known in the art.

In the compounds of this invention the term "lower alkyl" as used herein designates both straight and branched chain alkyl groups having 1 to 7 carbon atoms. Among the preferred lower alkyl groups are methyl, ethyl, isopropyl, n-butyl, etc., with methyl and ethyl being especially preferred.

In accordance with the invention the compounds of formula I can be prepared by reacting a compound of formula

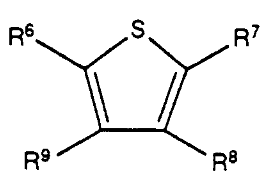

II

wherein either

$R^6$ is hydrogen or lower alkyl,
$R^7$ is $-CH_2-P(Ph)_3Hal$,
$R^8$ is $-O(CH_2)_nCH_3$, and
$R^9$ is hydrogen; or
$R^6$ is $-O(CH_2)_nCH_3$,

$R^7$ is $-CH_2P(Ph)_3Hal$, and
$R^8$ and $R^9$ are independently hydrogen or lower alkyl; or
$R^6$ and $R^7$ are independently hydrogen or lower alkyl,
$R^8$ is $-CH_2P(Ph)_3Hal$, and
$R^9$ is $-O(CH_2)_nCH_3$;
Ph is Phenyl; Hal is halogen; and n is an integer from 3 to 8; with a compound of formula

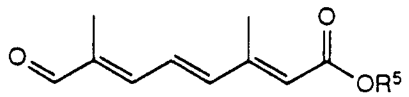

III

wherein $R^5$ is as defined above and, if desired, converting an ester group $OR^5$ into an hydroxy group or an amide or N-lower alkyl-amide group,

or a salt.

The reaction of the compound of formula II with the compound of formula III can be carried out under the conditions of the Wittig reaction, e.g. in the presence of butylene oxide at reflux or with more conventional bases such as alkyllithium reagents (n-butyllithium) or alkali metal alkoxides (sodium ethoxide).

Preferably, compounds of formula III wherein $R^5$ is lower alkyl are used for the reaction with the compound of formula II. In the compounds of formula II halogen is preferably chlorine or bromine.

The conversion of lower alkyl ester group $R^5$ into a carboxylic acid or amide can be accomplished by methods known per se, e.g. by hydrolysis with bases and, for the formation of an amide, converting the carboxylic acid group into a halogenide, such as the chloride and reacting the latter with the desired amine.

The starting compounds of formula II can be prepared by procedures as shown in Figures 1-3 below. In Figure 1, R is lower alkyl and X is halogen. $R^1$ and $R^2$ are hydrogen or lower alkyl

# Figure 1

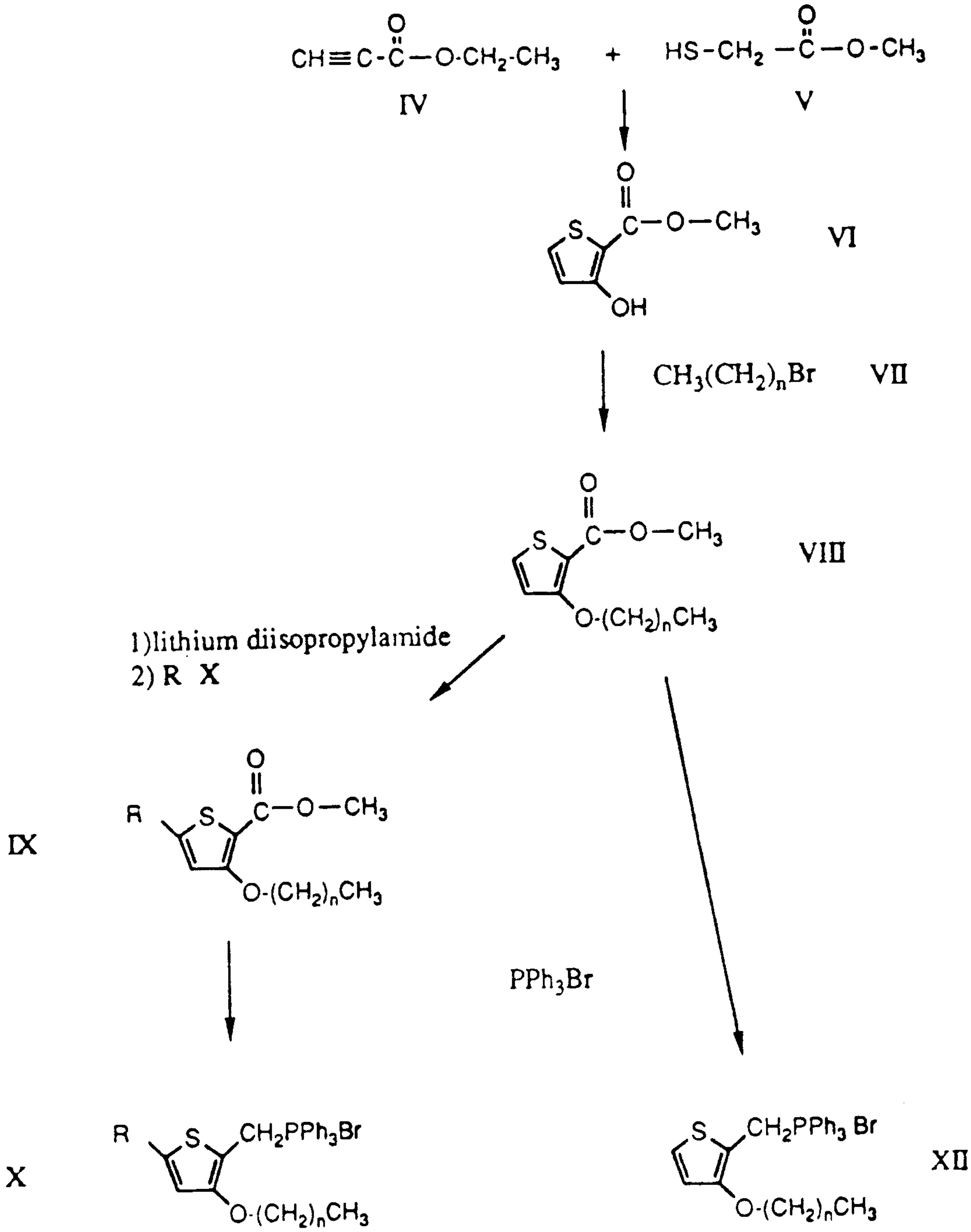
IV
V
VI
$CH_3(CH_2)_nBr$ VII
VIII
1)lithium diisopropylamide
2) R X
IX
$PPh_3Br$
X
XII

# Figure 2

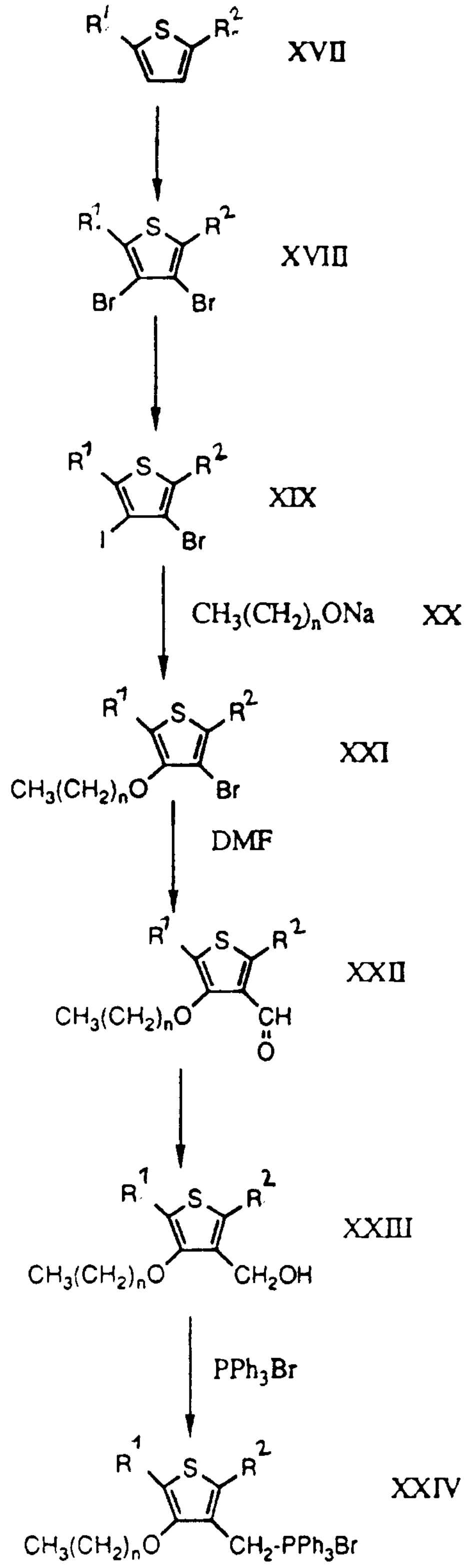


XVII

XVIII

XIX

XX

XXI

XXII

XXIII

XXIV

## Figure 3

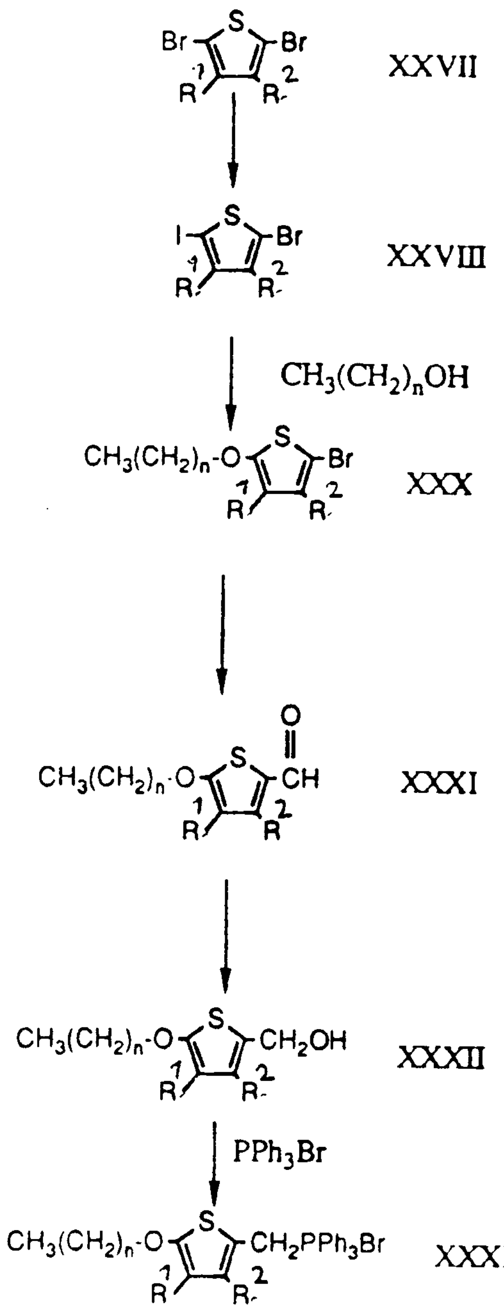


XXVII

XXVIII

XXIX

XXX

XXXI

XXXII

XXXIII

In the reaction scheme of Fig. 1, the compound of formula IV is reacted first with sodium methoxide and then with the compound of formula V to produce the compound of formula VI. The compound of formula VI is reacted with the n-alkyl bromide of formula VII wherein n may be from 3 to 10 to obtain the compound of formula VIII. If a compound of formula I is desired wherein $R^1$ is lower alkyl rather than hydrogen, the compound of formula VIII is sequentially treated with lithium diisopropylamide and a lower-alkyl halide (RX) to obtain the compound of formula IX.

The alkyl-substituted compound of formula IX or the non-alkyl substituted compound of formula VIII undergo a form of the Wittig reaction with triphenylphosphonium hydrobromide to obtain the compounds of formulas X and XII, respectively.

In Fig. 2, selective metalation of the compound of formula XVIII with n-butyllithium followed by exposure

to iodine yields the 5-iodo-2-bromothiophene of formula XIX which on reaction with the compound of formula XX and copper oxide gives the compound of formula XXI. Transmetalation of this material with n-butyllithium and subsequent quenching with dimethylformamide then yields the aldehyde of formula XXII which after reduction to the alcohol of formula XXIII is converted to the phosphonium salt of formula XXIV with triphenylphosphonium hydrobromide.

Fig. 3 is identical in all material respects with the reaction scheme of Fig. 2.

The compounds of formula I, as well as salts thereof, are effective as disease modifiers for treating rheumatoid arthritis as well as relate autoimmune disorders. The activity of the compounds of formula I in these diseases may be related to their activity as immunosuppressants.

The compounds of formula I and salts thereof can be utilized to treat patients suffering from rheumatoid arthritis and related disorders. In such cases, the compounds modify the effects of these diseases by reducing destruction of the bone joints caused by the diseases as well as reducing inflammation, heat and pain of the bone joints which results from rheumatoid arthritis and related disorders. The compounds of formula I and salts thereof are also useful for treating diseases resulting from immune hyperactivity or inappropriate immune responses such as transplantation autoimmunity, autoimmune disease and graft versus host disease.

That the compounds of this invention are effective anti-arthritic agents can be seen from the results obtained when these compounds are administered to rats in accordance with the chronic adjuvant arthritis test system disclosed in Billingham and Davies "Handbook of Experimental Pharmacology" (editors J.R. Vane and S.H. Ferreira) Vol. 50/II, pg. 108-144, Springer-Verlag, Berlin, 1979).

In this procedure, adjuvant arthritis was induced by the injection on day 0 to 0.10 ml of adjuvant (a suspension of heat-killed, dessicated Mycobacterium butyricum, 0.5% (w/v), in heavy mineral oil containing 0.2% digitonin) subplantarly into the right hind paw ("developing" model) or the base of the tail ("established" model) of male Charles River Lewis rats (120-140 g) that were housed individually and given food and water ad lib. Paw volumes (both hind paws) were measured immediately after injection of the adjuvant. Paw volumes were also measured at selected time intervals to follow the development of inflammation-induced swelling in the arthritic paws by immersion of the paw to the level of the lateral malleolus in a mercury plethysmograph.

In the developing adjuvant arthritis model [Table 1], drugs were administered once a day (starting on the day of adjuvant injection and continuing for 23 days) by intubation using Tween 80 (polyoxyethylene sorbitan mono-oleate) at a dose of 0.25 ml/100 g body weight as the vehicle. Arthritic control rats received daily doses of the vehicle only. On day 23, i.e., approximately 24 hours after the last dose of drug, the rats were sacrificed, plasma was collected and plasma fibrinogen levels were determined (ammonium sulfate turbidimetric method) as described by Exner et al., Amer. J. Clin. Path., 71:521-527 (1979). Anti-inflammatory activity of the test drugs were determined by comparing the extent of paw swelling (paw volume on day 23 minus the paw volume on day 0) in drug-treated arthritic rats with the extent of paw swelling in the vehicle-treated arthritic rats. Drug induced decreases in the level of plasma fibrinogen, an acute phase protein that is elevated in the plasma of rats with adjuvant-induced arthritis, were also used to quantitiate the anti-inflammatory activity.

In the established adjuvant arthritis model [Table 2], the arthritis was allowed to develop untreated for 21 days and then the arthritic animals were divided into experimental groups with approximately equal mean hind paw volumes. Starting on day 21, the arthritic animals were treated by intubation once a day with drugs for 7 days (Tween 80 as vehicle). Arthritic control animals received daily doses of the vehicle only. On day 28, the animals were sacrificed, plasma was collected and plasma fibrinogen levels were determined as described above. Antiinflammatory activity was determined by comparing the change in paw swelling in drug-treated arthritic animals (combined hind paw volume on day 28 minus combined hind paw volume on day 21) with the change in paw swelling in vehicle-treated arthritic animals. Drug-induced changes in plasma fibrinogen were also used to assess the antiinflammatory effects of the test drugs.

Normal animals were also treated with the test retinoids for 14 days and serum samples were collected for the determination of serum triglycerides and cholesterol. Triglyceride and cholesterol levels in the drug-treated animals were compared with triglyceride and cholesterol levels in vehicle-treated animals.

The results of various compounds of this invention when compared to 4-hydroxyphenyl retinamide are given in the following Tables 1 and 2. The major feature of this invention is that these compounds are well tolerated and do not display the characteristic hypervitaminoisis A toxicity at a therapeutically effective dose (e.g., $ED_{50}$ in developing adjuvant arthritis). Also the hypertriglyceridemia and serum cholesterol elevation observed with other retinoids, e.g., 4-hydroxyphenyl retinamide, is not observed.

Table 1
Developing Adjuvant Arthritis Model(a)

| Structure | Dose (μ Moles/Kg) Po. Tween 80 | % Change in Paw(b) Volume (day 23) right | left | % Change in Plasma fibrinogen(b) | Body Weight(c) gain(g) | Triglycerides(d) (% change) | Cholesterol(d) (% change) |
|---|---|---|---|---|---|---|---|
| 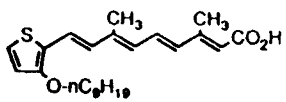 | 25 | -50* | -50* | -45* | 31(37) | + 5 | -14* |
| | 75 | -68* | -84* | -55* | 27(30) | + 21 | -25* |
| 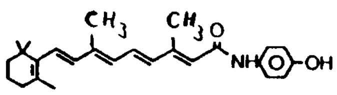 | 75 | -49* | -51* | -42* | 39(37) | + 66* | +66* |
| 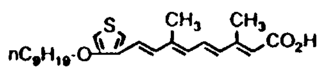 | 75 | -48* | -64* | -72* | 32*(43) | | |
| 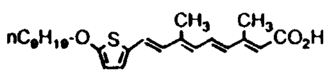 | 75 | -28* | -38* | -16* | 34*(43) | | |

a) Arthritis was induced by the injection of adjuvant into the right hind foot pad on day 0. Drugs were administered daily for 23 days.
b) Percent change in paw volume of plasma fibrinogen as compared with values in vehicle-treated arthritic animals.
c) Body weight gains equals body weight on day 23 minus body weight on day 0. The value in parenthesis is the weight gain for vehicle-treated arthritic animals.
d) Percent change in serum triglycerides and cholesterol in normal rats as compared with values for vehicle-treated normal rats.

*) These values represent significant changes from the values for vehicle-treated arthritic animals (Students t-test, $p<0.0$)

Table 2
Established Adjuvant Arthritis Model (a)

| Structure | Dose (µMoles/Kg) | Change in Paw Volume(b) (ml) | % Change in plasma fibrinogen(c) | Change in body weight(g)(b) |
|---|---|---|---|---|
| 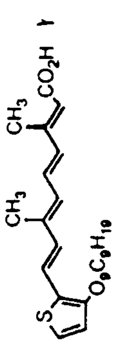 | 25 p.o.<br>75, p.o.<br>tween 80 | 0.15*(0.84)<br>-0.63*(0.61) | -32*<br>-34* | 9(14)<br>5*(15) |
| 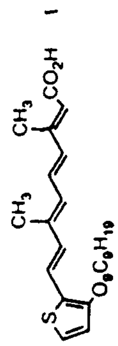 | 10 i.p.<br>corn oil | 0.18* (0.86) | -18* | 11(15) |
| 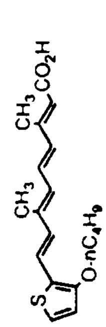 | 75 i.p.<br>corn oil | -0.59* (1.14) | -55* | 11(10) |
| 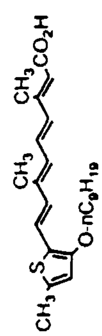 | 75 p.o.<br>tween 80 | 0.06* (1.03) | -25* | 15(14) |

a) Arthritis was induced by the injection of adjuvant into the base of the tail on day 0. On day 21, the animals were divided into groups with approximately equal mean hind paw volume and drug therapy was initiated. Drug therapy was continued for 7 days.

b) Change in paw volume/body weight equals the difference from day 21 to day 28; the values in parentheses are for vehicle treated arthritic animals.

c) Percent change in plasma fibrinogen relative to ..a value for vehicle-treated arthritic animals.

* These values represent significant changes from the values for vehicle-treated arthritic animals (Student's t-test, $p<0.05$).

In the above Table 1, the percent reduction in paw volume demonstrates the effectiveness of compounds of this invention to supress swelling caused by adjuvant arthritis when the compounds were administered starting on day 0, i.e., the day that adjuvant was injected to induce the arthitis. Furthermore, the compounds of this invention were effective in reducing the plasma fibrinogen generally associated with inflammation. Furthermore, as seen from the weight gain of the animals, the compounds of this invention at the dosage tested produced no substantial reduction in the weight gain of the animals. This indicated the lack of toxicity exhibited by the compounds of this invention.

The data in the above Table 2 demonstrates that the compounds of this invention are also effective as therapeutic agents when used to treat established adjuvant arthritis. The data on changes in paw volume and fibrinogen demonstrate that the compounds retard the progression of established arthritis with no evidence of toxicity as reflected by the lack of difference in weight gain between drug and vehicle treated animals.

From this series of compounds the most preferred material is (all-E)-3,7-dimethyl-9-[3-(nonyloxy)-2-thienyl]-2,4,6,8-nonatetraenoic acid.

The compounds of formula I and their pharmaceutically acceptable salts can be used in a variety of

pharmaceutical preparations. In these preparations, these compounds are administrable in the form of oral unit dosage forms such as tablets, pills, powders, and capsules, as well as in such forms such as injectables, solutions, suppositories, emulsions, dispersions, and in other suitable forms. The pharmaceutical preparations which contain the compounds of formula I are conveniently formed by admixing the compounds with a non-toxic pharmaceutical organic carrier or a pharmaceutically acceptable carriers are, for example, water, gelatin, lactose, starches, magnesium stearate, talc, vegetable oils, polyalkylene glycols, petroleum jelly and other conventionally employed pharmaceutically acceptable carriers. The pharmaceutical preparations may also contain non-toxic auxiliary substances such as emulsifying, preserving and wetting agents and the like, as for example, sorbitan monolaurate, triethanolamine oleate, polyoxyethylene sorbitan, dioctyl sodium sulfosuccinate and the like.

The daily dose administered for the compounds will, of course, vary with the particular novel compound employed, the chosen route of administration and the size of the recipient. The dosage administered is not subject to definite bounds but it will usually be in effective amounts of the pharmacologically function of the compounds of this invention. Representative of a typical method for administering the compounds of formula I or their salts is by oral administration. By this route, the compounds of formula I, or their salts can be administered at a dosage of 0.5 mg/kg per day p.o. to 100 mg/kg per day p.o. Preferably these compounds can be administered daily to patients in unit oral dosage forms at daily dosages of from 1 to 30 mg/kg of body weight, with dosages of from 1 to 10 mg/kg being especially preferred.

The invention is illustrated further by the Examples given below.

Example 1

A suspension of potassium carbonate (60 g), methyl-3-hydroxy-2-thiophenecarboxylate (38 g) and nonyl bromide (54 g) in dimethylformamide (DMF) was heated at 75-80°C for 48 hours. The reaction was cooled, poured into water and extracted into hexane. The hexane extract was washed with water, dried ($MgSO_4$) and evaporated. The product was crystallized from hexane to give methyl-3-nonyloxy-2-thiophenecarboxylate.

Employing n-butyl bromide in the above procedure yielded methyl-3-butyloxy-2-thiophenecarboxylate.

Example 2

A solution of methyl-3-nonyloxy-2-thiophenecarboxylate (22 g) in THF (900 ml) at -40°C was treated with diisobutylaluminum hydride (77 ml). The temperature was raised to -10°C and the reaction treated with 20 ml of saturated aqueous $Na_2SO_4$ and stirred at 30-32°C for 14 hours. The solution was diluted with 500 ml of hexane, treated with 50 g of $MgSO_4$, and filtered through sand. The mixture evaporated to about 250 ml, diluted with hexane, washed with water and brine and dried with $MgSO_4$. The solvent was evaporated to give 3-nonyloxy-2-thiophenemethanol which was used in the next step without further purification.

Reduction of methyl-3-n-butyloxy-2-thiophenecarboxylate in a like fashion yielded 3-butyloxy-2-thiophenemethanol.

Example 3

3-Nonyloxy-2-thiophenemethanol (17 g) and triphenylphosphonium hydrobromide (24 g) were combined with acetonitrile (200 ml) and refluxed for 20 hours. The solvent was evaporated and the glass-like product was dried over $P_2O_5$ to yield the phosphonium salt.

In a similar manner [3-[(butoxy)thiophene]-2-methyl]triphenylphosphonium bromide was prepared from 3-butoxy-2-thiophenemethanol.

Example 4

A suspension of [3-[(nonyloxy)thiophene]-2-methyl]triphenylphosphonium bromide (39 g) in butylene oxide (100 ml) and toluene (400 ml) was treated with (all-E)-ethyl-3-methyl-7-formyl-2,4,6-octatrienoate (14 g) and refluxed under argon for 6 hours. All solvents were evaporated off and the residue was dissolved in hexane. The hexane solution was washed with 60% methanol/$H_2O$ solution, water, dried ($MgSO_4$), and evaporated to give an orange solid. This was purified by silica gel chromatography (10% ethyl ether/hexane) to give an oil which was crystallized from hexane yielding (all-E)-ethyl-3,7-dimethyl-9-[3-(nonyloxy)-2-thienyl]-2,4,6,8-nonatetraenoate (14 g).

In a similar manner (all-E)-ethyl-3,7-dimethyl-9-[3-(butyloxy)-2-thienyl]-2,4,6,8-nonatetraenoate was prepared from [3-[(butoxy)thiophene]-2-methyl]-triphenylphosphonium bromide.

Example 5

(all-E)-Ethyl-3,7-dimethyl-9-[3-(nonyloxy)-2-thienyl]-2,4,6,8-nonatetraenoate (5.5 g) was dissolved in ethanol (150 ml) and treated with a solution of potassium hydroxide (6 g/40 ml water). The reaction was warmed to 80 °C for 3 hours and monitored for the disappearance of the starting ester. The reaction was cooled and poured into 2N HCl and extracted into chloroform. The chloroform was washed with water, dried ($MgSO_4$) and evaporated to give a solid. This was crystallized from ethyl acetate/hexane go give pure (all-E)-3,7-dimethyl-9-[3-(nonyloxy)-2-thienyl]-2,4,6,8-nonatetraenoic acid (2.3 g); mp 139-140 °C.

In a similar manner (all-E)-3,7-dimethyl-9-[3-(butyloxy)-2-thienyl]-2,4,6,8-nonatetraenoic acid was prepared from (all-E)-ethyl-3,7-dimethyl-9-[3-(butyloxy)-2-thienyl]-2,4,6,8-nonatetraenoate.

Example 6

A solution of 3,4-dibromothiophene (160 g) in ether (1.5 l) at -75 °C was treated with n-butyllithium (440 ml). This mixture was then treated with a solution of iodine (178 g) in THF (400 ml). The reaction was warmed to - 20 °C and poured into water and extracted with hexane. The hexane phase was washed with 5% sodium thiosulfate, water, dried over $MgSO_4$ and evaporated. All low boiling material (<125 °C/0.05 mm) was distilled off and the pot residue (3-iodo-4-bromothiophene) was pure enough for the next step.

Example 7

A solution of 1-nonanol (400 g) in 400 ml of dimethoxyethane (DME) was treated with sodium hydride (washed free of oil) (48 g) in portions and warmed at 60-65 °C for 1 hour. Copper oxide (40 g) was added followed by 3-iodo-4-bromothiophene (145 g). The mixture was stirred at 60 °C for 4 days. The reaction was filtered through sand-celite and extracted well with ether. The ether layer was washed with water, dried ($MgSO_4$) and fractionated yielding 3-nonyloxy-4-bromothiophene, b.p. 126-140 °C/0.01 mm.

Example 8

A solution of n-butyllithium (1.5M, 240 ml) in hexane was added to 3-nonyloxy-4-bromothiophene (110 g) in 1.5 l of ether at -78 °C. After being stirred at -70 °C for 15 minutes, a solution of DMF (40 g) in 100 ml of ether was added. After 15 min, the reaction was poured into water and the product extracted into hexane. The hexane phase was washed with water, dried ($MgSO_4$) and evaporated to give 4-nonyloxy-3-thiophenecarboxaldehyde which was used directly in the next step.

Example 9

A solution of 4-nonyloxy-3-formylthiophene (89 g) in 500 ml of ethanol at 10 °C was treated with sodium borohydride (6 g) and stirred at 23 °C for 2 hours. The reaction was poured into water and the product extracted into hexane. The hexane phase was washed with water, dried ($MgSO_4$) and evaporated to give an oil. Crystallization from hexane gave 38 g of 4-nonyloxy-3-thiophenemethanol.

Example 10

4-Nonyloxy-3-thiophenemethanol (34 g) and triphenylphosphonium bromide (48 g) were combined in acetonitrile and warmed to 80 °C for 20 hours. The reaction was cooled and triturated with ether to precipitate the salt. After filtration and drying over $P_2O_5$, pure [4-[(nonyloxy)thiophene]-3-methyl]-triphenylphosphonium bromide was obtained as a white solid.

Example 11

A 1.6M n-butyllithium solution (94 ml) was added to [4-[(nonyloxy)-thiophene]-3-methyl]-triphenylphosphonium bromide in 1 liter of THF at -75 °C. This was then treated with ethyl (all-E)-3-methyl-7-formyl-2,4,6-octatrienoate (34 g) in 100 ml THF and the reaction was warmed to -40 °C. The reaction was treated with 20 ml of acetic acid, poured into water and the product was extracted into hexane. The hexane phase was washed with water, 60% methanol/$H_2O$, and brine. Alter drying ($MgSO_4$) and evaporation, the crude product was partially purified by silica gel chromatography (10% ether/hexane). Crystallization from hexane gave ethyl (all-E)-3,7-dimethyl-9-[4-(nonyloxy)-3-thienyl]-2,4,6,8-nonatetraenoate (24 g).

Example 12

Ethyl (all-E)-3,7-dimethyl-9-[4-(nonyloxy)-3-thienyl]-2,4,6,8-nonatetraenoate (6 g) dissolved in 200 ml of ethanol was hydrolyzed with 10% KOH (40 ml) as in Example 5. Acidification and work-up was similar to Example 5, and crystallization from cold ethyl acetate gave pure (all-E)-3,7-dimethyl-9-[4-(nonyloxy)-3-thienyl]-2,4,6,8-nonatetraenoic acid (4 g); mp 133-134 ° C.

Example 13

A solution of 2,5-dibromothiophene (100 g) in 1 liter of ether was metalated with 1.6M n-butyllithium (270 ml) and iododinated with iodine (110 g) in 200 ml of THF as described in Example 6 to yield 5-iodo-2-bromothiophene.

Example 14

A suspension of sodium nonanate (0.81 mol) in 800 ml of dimethoxyethane was prepared as in Example 7 with sodium hydride (41 g) and 1-nonanol (115 g). Copper oxide (0.5 mol) and 2-iodo-5-bromothiphene (118 g) were added and the mixture heated at 80 ° C for 20 hours. Work-up as in Example 7 followed by silia gel purification gave pure 2-nonyloxy-5-bromothiophene.

Example 15

A solution of 2-nonyloxy-5-bromothiophene (50 g) in 800 ml of ether was metalated with 1.6M n-butyllithium (103 ml) and treated with DMF (20 g) in 100 ml of THF as in Example 8. Low temperature crystallization from ether/hexane gave 5-nonyloxy-2-formylthiophene (oil at room temperature) which was reduced directly without further purification in the next step.

Example 16

A solution of 5-nonyloxy-2-thiophenealdehyde (35 g crude) was reduced with sodium borohydride in a manner similar to Example 9. This gave 5-nonyloxy-2-thiophenemethanol after crystallization from ether/hexane.

Example 17

A mixture of 5-nonyloxy-2-thiophenemethanol (11 g) and triphenylphosphonium bromide (165 g) in 200 ml of acetonitrile was warmed to 80 ° C for 16 hours. The solvent was removed in vacuo and the residue was dried at 40 ° C/0.1 mm to give [5-[(nonyloxy)thiophene]-2-methyl]triphenylphosphonium bromide of sufficient purity to be used in the next step.

Example 18

The salt from Example 17 ([5-(nonyloxy)thiophene-2-methyl]triphenylphosphonium bromide, 25 g) was converted to the title compound as described in Example 11. Purification of the reaction mixture by silica gel chromatography followed by crystallization from hexane yielded pure ethyl-(all-E)-3,7-dimethyl-9-[5-(nonyloxy)-2-thienyl]-2,4,6,8-nonatetraenoate (11.6 g); mp 75-76 ° C.

Example 19

Ethyl-(all-E)-3,7-dimethyl-9-[5-(nonyloxy)-2-thienyl]-2,4,6,8-nonatetraenoate (5 g) was hydrolysed as described in Example 5. Crystallization of the crude acid from a hexane/ethyl acetate mixture yielded pure (all-E)-3,7-dimethyl-9-[5-(nonyloxy)-2-thienyl]-2,4,6,8-nonatetraenoic acid (3 g); mp 150-154 ° C.

Example 20

A solution of methyl thioglycolate (106 g) in methanol (2 liters) was treated with 850 ml of sodium methoxide in methanol (2.45M) while being cooled at 10-15 ° C. The solution was cooled to -10 ° C and treated with ethyl propiolate (100 g; 1.04 mol). The solution was stirred at 5 ° C for 0.5 hours before raising

the temperature to 40-42 °C for 4 hours. Partial evaporation of the solvent mixture in vacuo left a gummy residue of the sodium salt of 2-carbomethoxy-3-hydroxythiophene. This residue was decanted into a concentrated hydrochloric acid-ice slurry and extracted with chloroform three times. The chloroform solution was washed with an ice-cold solution of a 1:1 mixture of brine-water. The chloroform solution was dried ($Na_2SO_4$), treated with norite A and filtered through celite. Evaporation of the solvent in vacuo gave a light brown gum which was dissolved in hexane (2 liters), cooled to 15 °C, treated with norite A and filtered through celite to give a light yellow solution. The product was crystallized at -20 °C. Filtration gave 97 g of 2-carbomethoxy-3-hydroxythiophene.

Example 21

A solution of 2-carbomethoxy-3-hydroxythiophene (94 g) in methylethyl ketone (1.3 liters) was treated with powdered potassium carbonate (138 g), 1-bromononane (125 g) and dimethylformamide (100 ml). The mixture was heated at reflux for 20 hours. The suspension was filtered through a sintered glass funnel and the solution was diluted with cold water (4 liters) with stirring. The solids were filtered and crystallized from ethanol at -20 °C. This gave 153 g of 3-(nonyloxy)-2-thiophenecarboxylic acid methyl ester.

Example 22

A solution of 2-carbomethoxy-3-nonyloxythiophene (21 g) was dissolved in toluene (400 ml), cooled to -40 °C and treated with diisobutylaluminum hydride (150 ml of 1.0M solution in hexane). The solution was warmed to 0 °C and treated with 50 ml of a solution of saturated sodium sulfate and stirred at ambient temperature for 2 hours. This suspension was filtered through a bed of sand-celite and the solvent was removed in vacuo below 40 °C. The remaining oil was crystallized from hexane to give 16.5 g of 3-(nonyloxy)-2-thiophenemethanol. The compound is acid sensitive and must be stored at -20 °C.

Example 23

A solution of triphenylphosphonium hydrobromide (22 g) in methylene chloride (500 ml) was treated with powdered sodium sulfate and cooled to -30 °C. With good stirring, a solution of 3-(nonyloxy)-2-thiophenemethanol (16.4 g) in methylene chloride (100 ml) was added and the reaction was allowed to reach ambient temperature (1 hour). The reaction mixture was filtered through celite and the solvent was evaporated in vacuo. The remaining gum was crystallized from a mixture of tetrahydrofuran/ether go give 28 g of [2-(3-nonyloxy)thienyl)methyl]triphenylphosphonium bromide.

Example 24

A mixture of finely powdered [2-(3-nonyloxy)thienyl)methyl]triphenylphosphonium bromide (14 g) and (all-E)-3,7-dimethyl-7-formyl-2,4,6-heptatrienoic acid ethyl ester (5.1 g) was suspended in THF (400 ml). The mixture was cooled to -20 °C and sodium ethoxide in ethanol (1.12M; 22 ml) was added. The reaction was allowed to stir at ambient temperature for 1 hour. The mixture was diluted with water and extracted with hexane. The hexane extract was washed with water, a 60% methanol/water solution, dried ($Na_2SO_4$), and concentrated to give crude (all-E)-3,7-dimethyl-9-[3-nonyloxy)-2-thienyl]-2,4,6,8-nonatetraenoic acid ethyl ester. This was partially purified by chromatography (silica gel; 5% ethyl acetate/hexane) to give an oil. The product was crystallized from hexane.

Example 25

A solution of (all-E)-3,7-dimethyl-9-[3-(nonyloxy)-2-thienyl]-2,4,6,8-nonatetraenoic acid ethyl ester (1.7 g) in ethanol (200 ml) was treated with a solution of potassium hydroxide (2 g) in water (40 ml). This was refluxed for 1.5 hours. The solution was cooled and decanted into dilute aqueous phosphoric acid. The acidic mixture was extracted with chloroform and the chloroform extract was washed with water, dried ($Na_2SO_4$), filtered, and concentrated to an orange oil. The product was crystallized from THF/hexane to give 1.1 g of (all-E)-3,7-dimethyl-9-[3-(nonyloxy)-2-thienyl]-2,4,6,8-nonatetraenoic acid.

Example 26

A solution of 3-(nonyloxy)-2-thiophenecarboxylic acid methyl ester (11.4 g) in THF (50 ml) was added to

a freshly prepared solution of lithium diisopropylamide (LDA) (43 mmol) in THF (400 ml) at -70 ° C. This mixture was stirred for 10 minutes and subsequently treated with methyl iodide (7.1 g) and warmed to -20 ° C. The mixture wa diluted with water and extracted into hexane. The extracts were washed with water, dried ($Na_2SO_4$), and concentrated to give 11 g of 5-methyl-3-nonyloxy-2-thiophenecarboxylic acid methyl ester.

Example 27

5-Methyl-3-nonyloxy-2-thiophenecarboxylic acid methyl ester was conveted into 5-methyl-3-nonyloxy-2-thiophene-methanol in a manner similar to that described in Example 2 for the preparation of the demethylated thiophene.

Example 28

A solution of triphenylphosphonium bromide (15.4 g), 5-methyl-3-nonyloxy-2-thiophene-methanol (5.6 g) and acetonitrile (300 ml) was refluxed for 3 hours. The solvent was removed in vacuo and [2-(5-methyl-3-nonyloxy)-thienyl)methyl]triphenylphosphonium bromide was dried under high vacuum. The salt was dissolved in THF and used in the succeeding step.

Example 29

A solution of [2-(5-methyl-3-nonyloxy)thienyl)methyl]triphenylphosphoniumbromide (21 g) in THF (400 ml) was cooled to -70 ° C and treated with n-butyllithium (28.2 ml; 1.6M in hexane). The temperatur was raised to -20 ° C for 10 min and they cooled to -60 ° C. The orange ylide thus obtained was tgreated with (all-E)-3,7-dimethyl-7-formyl-2,4,6-heptatrienoic acid ethyl ester in THF (50 ml). The temperatur was allowed to reach ambient over about 1 hour. The reaction mixture was then diluted with water and extracted with hexane. The hexane extracts were washed with water and with a 60% methanol/water solution. After drying ($Na_2SO_4$) and filtration, the solvent was removed in vacuo and the red residue was partially purified by chromatography on silica gel (5% ethyl acetate/hexane) to give an oil. Crystallization from hexane gave 6 g of purple (all-E)-3,7-dimethyl-9-[5-methyl-3-nonyloxy)-2-thienyl]-2,4,6,8-nonatetraenoic acid ethyl ester.

The acid was obtained by hydrolysis of the ester in a manner similar to Example 25.

**Claims**

**1.** A compound of the formula

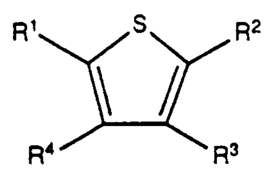

I

wherein either

| | |
|---|---|
| $R^1$ | is hydrogen or lower alkyl, |
| $R^2$ | is a moiety Z, |
| $R^3$ | is $-O(CH_2)_nCH_3$, and |
| $R^4$ | is hydrogen; or |
| $R^1$ | and $R^2$ are independently hydrogen or lower alkyl, |
| $R^3$ | is a moiety Z, and |
| $R^4$ | is $-O(CH_2)_nCH_3$; or |
| $R^1$ | is $-O(CH_2)_nCH_3$, |
| $R^2$ | is a moiety Z, and |
| $R^3$ | and $R^4$ are independently hydrogen or lower alkyl; |
| Z | is a moiety |

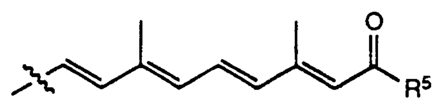

$R^5$ is hydrogen, lower alkyl, amino or N-lower alkylamino; and n is an integer from 3 to 8; and salts of compounds of formula I wherein $R^5$ is hydrogen.

**2.** A compound of claim 1 wherein $R^1$ is hydrogen or lower alkyl; $R^2$ is a moiety Z; $R^3$ is $-O(CH_2)_nCH_3$; $R^4$ is hydrogen; and n and Z are as in claim 1.

**3.** The compound of claim 2 wherein said compound is (all-E)-3,7-dimethyl-9-[3-(nonyloxy)-2-thienyl]-2,4,6,8-nonatetraenoic acid.

**4.** The compound of claim 2 wherein said compound is (all-E)-3,7-dimethyl-9-[3-(butyloxy)-2-thienyl]-2,4,6,8-nonatetraenoic acid.

**5.** A compound of claim 1 wherein $R^1$ and $R^2$ are, independently, hydrogen or lower alkyl; $R^3$ is a moiety Z; $R^4$ is $-O(CH_2)_nCH_3$; and n and Z are as in claim 1.

**6.** The compound of claim 5 wherein said compound is (all-E)-3,7-dimethyl-9-[4-(nonyloxy)-3-thienyl]-2,4,6,8-nonatetraenoic acid.

**7.** A compound of claim 1 wherein $R^1$ is $-O(CH_2)_nCH_3$; $R^2$ is a moiety Z; $R^3$ and $R^4$ are independently hydrogen or lower alkyl; and n and Z are as in claim 1.

**8.** The compound of claim 7 wherein said compound is (all-E)-3,7-dimethyl-9-[5-(nonyloxy)-2-thienyl]-2,4,6,8-nonatetraenoic acid.

**9.** A compound of any one of claims 1-8 for use as a medicament.

**10.** Process for the preparation of a compound of formula I as defined in claim 1 or a salt thereof which process comprises reacting a compound of formula

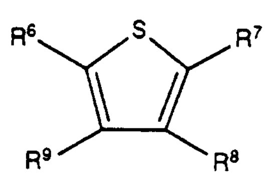

II

wherein either

$R^6$ is hydrogen or lower alkyl,
$R^7$ is $-CH_2-P(Ph)_3Hal$,
$R^8$ is $-O(CH_2)_nCH_3$, and
$R^9$ is hydrogen; or
$R^6$ is $-O(CH_2)_nCH_3$,
$R^7$ is $-CH_2P(Ph)_3Hal$, and
$R^8$ and $R^9$ are independently hydrogen or lower alkyl; or
$R^6$ and $R^7$ are independently hydrogen or lower alkyl,
$R^8$ is $-CH_2P(Ph)_3Hal$, and
$R^9$ is $-O(CH_2)_nCH_3$;
Ph is Phenyl; Hal is halogen; and n is an integer from 3 to 8; with a compound of formula

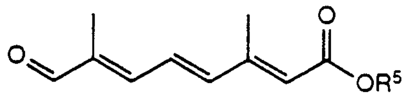

III

wherein $R^5$ is a defined in claim 1 and, if desired, converting an ester group $OR^5$ into an hydroxy group or an amide or N-lower alkylamide group,

or a salt.

**11.** Pharmaceutical compositions containing a compound of formula I as defined in claim 1 or a salt thereof, and a pharmaceutical carrier.

**12.** The use of a compound of formula I as defined in claim 1 or a salt thereof in the manufacture of pharmaceutical compositions for the treatment of psoriatic or rheumatoid arthritis.

**Claims for the following Contracting States: GR, ES**

**1.** Process for the preparation of compounds of the formula

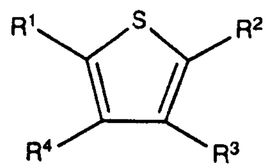

I

wherein either

$R^1$ is hydrogen or lower alkyl,
$R^2$ is a moiety Z,
$R^3$ is $-O(CH_2)_nCH_3$, and
$R^4$ is hydrogen; or
$R^1$ and $R^2$ are independently hydrogen or lower alkyl,
$R^3$ is a moiety Z, and
$R^4$ is $-O(CH_2)_nCH_3$; or
$R^1$ is $-O(CH_2)_nCH_3$,
$R^2$ is a moiety Z, and
$R^3$ and $R^4$ are independently hydrogen or lower alkyl;
Z is a moiety

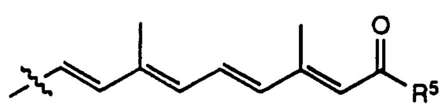

$R^5$ is hydrogen, lower alkyl, amino or N-lower alkylamino; and n is an integer from 3 to 8;

and salts of compounds of formula I wherein $R^5$ is hydrogen, which process comprises reacting a compound of formula

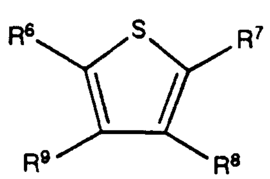

II

wherein either

$R^6$ is hydrogen or lower alkyl,

$R^7$ is $-CH_2-P(Ph)_3Hal$,
$R^8$ is $-O(CH_2)_nCH_3$, and
$R^9$ is hydrogen; or
$R^6$ is $-O(CH_2)_nCH_3$,
$R^7$ is $-CH_2P(Ph)_3Hal$, and
R and $R^9$ are independently hydrogen or lower alkyl; or
$R^6$ and $R^7$ are independently hydrogen or lower alkyl,
$R^8$ is $-CH_2P(Ph)_3Hal$, and
$R^9$ is $-O(CH_2)_nCH_3$;
Ph is Phenyl; Hal is halogen; and n is an integer from 3 to 8; with a compound of formula

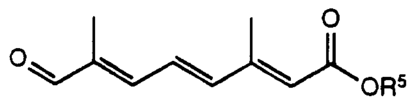

III

wherein $R^5$ is a defined in claim 1 and, if desired, converting an ester group $OR^5$ into an hydroxy group or an amide or N-lower alkylamide group,

or a salt.

2. The process of claim 1 for the preparation of compounds of claim 1 wherein $R^1$ is hydrogen or lower alkyl; $R^2$ is a moiety Z; $R^3$ is $-O(CH_2)_nCH_3$; $R^4$ is hydrogen; and n and Z are as in claim 1.

3. The process of claim 1 for the preparation of (all-E)-3,7-dimethyl-9-[3-(nonyloxy)-2-thienyl]-2,4,6,8-nonatetraenoic acid.

4. The process of claim 1 for the preparation of (all-E)-3,7-dimethyl-9-[3-(butyloxy)-2-thienyl]-2,4,6,8-nonatetraenoic acid.

5. The process of claim 1 for the preparation of compounds of claim 1 wherein $R^1$ and $R^2$ are, independently, hydrogen or lower alkyl; $R^3$ is a moiety Z; $R^4$ is $-O(CH_2)_nCH_3$; and n and Z are as in claim 1 for the preparation of (all-E)-3,7-dimethyl-9-[4-(nonyloxy)-3-thienyl]-2,4,6,8-nonatetraenoic acid.

7. The process of claim 1 for the preparation of compounds of claim 1 wherein $R^1$ is $-O(CH_2)_nCH_3$; $R^2$ is a moiety Z; $R^3$ and $R^4$ are independently hydrogen or lower alkyl; and n and Z are as in claim 1.

8. The process of claim 1 for the preparation of (all-E)-3,7-dimethyl-9-[5-(nonyloxy)-2-thienyl]-2,4,6,8-nonatetraenoic acid.

European Patent Office

# EUROPEAN SEARCH REPORT

Application Number

EP 92 10 6339

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 230 872 (M. J. KLAUS ET AL.)<br>* the whole document *<br>--- | 1,9,11 | C07D333/32<br>A61K31/38 |
| X | FR-A-2 331 338 (F. HOFFMANN-LA ROCHE & CIE.)<br>* the whole document *<br>----- | 1,9,11 | |
| | | | **TECHNICAL FIELDS SEARCHED (Int. Cl.5)** |
| | | | C07D |

The present search report has been drawn up for all claims

| Place of search | Date of completion of the search | Examiner |
|---|---|---|
| THE HAGUE | 29 JULY 1992 | B. Paisdor |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document